# EUROPÄISCHE PATENTSCHRIFT

(11) **EP 0 510 448 B1**
(45) Veröffentlichungstag und Bekanntmachung des Hinweises auf die Patenterteilung: **01.03.2000**
(21) Anmeldenummer: 92106144.6
(22) Anmeldetag: 09.04.1992
(51) Int. Cl.: C07D 251/34, G03C 1/73

(54) **Sulfonsäureester von 2,4,6-Tris-(2-hydroxy-ethoxy)- 1,3,5 triazin, ein damit hergestelltes positiv arbeitendes strahlungsempfindliches Gemisch und Aufzeichnungsmaterial**
Sulfonic acid esters of 2,4,6-tris-(2-hydroxy-ethoxy)-1,3,5-triazine, radiation-sensitive positive functioning mixture containing them and recording material
Esters d'acide sulfonique de 2,4,6-tris-(2-hydroxy-ethoxy)-1,3,5-triazine, mélange photosensible les contenant à positionnement positif et matériau d'enregistrement

(30) Priorität: 20.04.1991 DE 4112971
(43) Veröffentlichungstag der Anmeldung: 28.10.1992
(73) Patentinhaber: Clariant GmbH, 65929 Frankfurt am Main (DE)
(72) Erfinder: Röschert, Horst, Dr., W-6531 Ober Hilbersheim (DE); Pawlowski, Georg, Dr., W-6200 Wiesbaden (DE); Fuchs, Jürgen, W-6093 Flörsheim/Wicker (DE)

(56) Entgegenhaltungen:
- EP-A- 0 165 608
- EP-A- 0 251 059
- DE-A- 3 930 086
- DE-A- 3 930 087

## Beschreibung

Die Erfindung betrifft Sulfonsäureester von 2,4,6-Tris-(2-hydroxy-ethoxy)-[1,3,5]triazin und ein damit hergestelltes positiv arbeitendes strahlungsempfindliches Gemisch mit
a) einer Verbindung, die unter Einwirkung von aktinischer Strahlung eine starke Säure bildet,
b) einer Verbindung mit mindestens einer durch Säure spaltbaren C-O-C- oder C-O-Si-Bindung und
c) einem in Wasser unlöslichen, in wäßrig-alkalischen Lösungen löslichen oder zumindest quellbaren polymeren Bindemittel.

Die Erfindung betrifft weiterhin ein damit hergestelltes strahlungsempfindliches Aufzeichnungsmaterial, das zur Herstellung von Photoresists, elektronischen Bauteilen, Druckplatten oder zum Formteilätzen geeignet ist.

Die stetige Verkleinerung der Strukturen, beispielsweise in der Chip-Herstellung bis in den Bereich von weniger als 1 µm, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwellige Strahlung angepaßt sein. Eine Zusammenstellung der an das strahlungsempfindliche Gemisch gestellten Forderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Gemische, die neben einem in Wasser unlöslichen, in wäßrig-alkalischen Lösungen löslichen oder zumindest quellbaren Bindemittel und einer unter Einwirkung von aktinischer Strahlung eine starke Säure bildenden Komponente als wesentlichen Bestandteil eine durch Säure spaltbare Verbindung, mit z. B. C-O-C- oder C-O-Si-Bindungen, aufweisen, sind prinzipiell bekannt, beispielsweise aus der DE-A 23 06 248 (= US-A 3 779 778).

Als Verbindungen, die bei Bestrahlung eine starke Säure bilden, wurden bisher insbesondere Onium-Salze, wie Diazonium-, Phosphonium-, Sulfonium- und Jodonium-Salze von nicht nucleophilen Säuren, wie HSbF₆, HAsF₆, oder HPF₆ [J.V. Crivello, Polym. Eng. Sci., 23 (1983) 953], verwendet. Daneben sind Halogenverbindungen, insbesondere Trichlormethyltriazin-Derivate oder Trichlormethyloxadiazol-Derivate, o-Chinondiazidsulfochloride, o-Chinondiazid-4-sulfonsäureester, Organometall-Organohalogen-Kombinationen, Bis(sulfonyl)diazomethane, Sulfonyl-carbonyl-diazomethane (DE-A 39 30 087) oder Nitrobenzyltosylate [F.M. Houlihan et al., SPIE Proc., Adv. in Resist Techn. and Proc. 920 (1988) 67] empfohlen worden.

Diese Verbindungen werden in negativ oder positiv arbeitenden strahlungsempfindlichen Gemischen verwendet. Die Verwendung derartiger photolytischer Säurebildner bringt aber gewisse Nachteile mit sich, die ihre Einsatzmöglichkeiten in verschiedenen Anwendungsbereichen drastisch einschränken. So sind z.B. viele der Oniumsalze toxisch. Ihre Löslichkeit ist in vielen Lösemitteln unzureichend, weshalb nur wenige Lösemittel zur Herstellung einer Beschichtungslösung geeignet sind. Darüber hinaus werden bei Verwendung der Oniumsalze z.T. unerwünschte Fremdatome eingeführt, die insbesondere in der Mikrolithographie zu Prozeßstörungen führen können. Ferner bilden die Oniumsalze bei der Photolyse sehr stark korrodierend wirkende Brönstedt-Säuren. Diese Säuren greifen empfindliche Substrate an, so daß der Einsatz solcher Gemische zu unbefriedigenden Ergebnissen führt. Wie bereits früher erwähnt, bilden auch die Halogenverbindungen sowie die Chinondiazidsulfonsäurechloride stark korrosiv wirkende Halogenwasserstoffsäuren. Ferner besitzen derartige Verbindungen auf bestimmten Substraten nur eine begrenzte Haltbarkeit, die dadurch verbessert wurde, daß zwischen Substrat und strahlungsempfindlicher, Verbindungen des Typs (a) enthaltender Schicht eine Zwischenschicht eingefügt wurde, was allerdings zu einer unerwünschten Zunahme von Defekten und zu einer verminderten Reproduzierbarkeit führte (DE-A 36 21 376 = US-A 4 840 867).

In neueren Arbeiten von F.M. Houlihan et al., SPIE 920, 67 (1988) wurden anhand positiv arbeitender Systeme gezeigt, daß neben den oben genannten Säurebildnern auch Nitrobenzyltosylate, die bei Belichtung Sulfonsäuren mit geringer Wanderungstendenz bilden, in bestimmten säurelabilen Resistformulierungen verwendbar sind. Es kann aus diesen Ergebnissen abgeleitet werden, daß solche Verbindungen auch für photohärtbare Systeme verwendet werden können. Die dabei erzielten Empfindlichkeiten und die thermische Stabilität der Photoresists erwiesen sich jedoch als unzureichend.

Bereits bekannt sind auch Resistformulierungen mit Naphthochinon-2-diazid-4-sulfonsäureestern, Oximsulfonaten, 1,2-Disulfonen, Bis-sulfonyl-diazomethan (DE-A 39 30 086) und Sulfonyl-carbonyl-diazomethan (DE-A 39 30 087). All diese Verbindungen bilden unter der Einwirkung aktinischer Strahlung nicht korrodierend wirkende Sulfonsäuren. Die photochemische Reaktion verläuft zudem mit einer unbefriedigenden Quantenausbeute. Die Resistformulierungen absorbieren Strahlung der Wellenlänge 248 nm in beträchtlichem Maße. Die Empfindlichkeit gegenüber Strahlung dieser Wellenlänge liegt im Bereich von 50 bis 100 mJ/cm². Strukturen einer Größenordnung von 0,5 µm und weniger lassen sich mit solchen Resists nicht abbilden.

Es ist ferner bekannt, mit Methan-, Ethan-, Propan-, Butan-, Benzol-, Toluol- oder Naphthalinsulfonsäure vollständig verestertes 1,2,3-Trihydroxy-benzol als photoaktiven Säurebildner in positiv arbeitenden Photoresistsystemen zu verwenden [T. Ueno et al., Chemical Amplification Positive Resist Systems Using Novel Sulfonates as Acid Generators, in "Polymers for Microelectronics - Science and Technology", Hrsg. Y. Tabata et al., Kodansha-Weinheim-New York, 1989, S. 66-67]. Diese Resistsysteme finden jedoch in der Praxis keine Verwendung, da ihre thermische Stabilität und Plasmaätzresistenz unzureichend ist und nach der Entwicklung Resistreste in den Gräben und nicht akzeptable Resistprofile zu beobachten sind.

Trotz der bisher geleisteten intensiven Forschungstätigkeit auf diesem Gebiet ist derzeit kein strahlungsempfindliches Gemisch bekannt, mit dem sich ein positiv arbeitendes strahlungsempfindliches Aufzeichnungsmaterial herstellen läßt, das eine hohe Empfindlichkeit im DUV-Bereich (200 bis 300 nm) sowie eine hohe Auflösung besitzt, bereits bei kurzzeitiger Bestrahlung eine ausreichende Menge einer nicht korrodierend wirkenden Säure freisetzt, die stark genug ist, um Verbindungen des Typs b) zu spalten und das zudem noch wäßrig-alkalisch entwickelbar ist.

Aufgabe der Erfindung war es daher, ein strahlungsempfindliches Gemisch auf Basis von säurebildenden in Kombination mit säurespaltbaren Verbindungen vorzuschlagen, wobei die photolytisch eine Säure bildende Verbindung (a) möglichst stabil auf allen bekannten Substraten sein sollte und als Photoprodukt eine nicht korrosiv wirkende Säure liefert.

Die Aufgabe wird gelöst durch ein strahlungsempfindliches Gemisch mit
a) einer Verbindung, die unter Einwirkung von aktinischer Strahlung eine starke Säure bildet,
b) einer Verbindung mit mindestens einer durch Säure spaltbaren C-O-C- oder C-O-Si-Bindung und
c) einem in Wasser unlöslichen, in wäßrig-alkalischen Lösungen löslichen oder zumindest quellbaren polymeren Bindemittel,
   das durch gekennzeichnet ist, daß die Verbindung a) ein mit zwei oder drei (Hetero)arylsulfonsäuren verestertes 2,4,6-Tris-(2-hydroxy-ethoxy)-[1,3,5]triazin ist.

Gegenstand der Erfindung ist auch ein mit zwei oder drei Aryl- und/oder Heteroarylsulfonsäuren verestertes 2,4,6-Tris-(2-hydroxyethoxy)-[1,3,5]triazin, wobei die Arylsulfonsäuren 6 bis 10 aromatische Kohlenstoffatome und die Heteroarylsulfonsäuren 4 bis 9 aromatische Kohlenstoffatome und entweder 1 aromatisches Sauerstoff- oder Schwefelatom oder 1 bis 2 aromatische Stickstoffatome aufweisen und wobei die Aryl- oder Heteroarylsulfonsäuren gegebenenfalls mit (C₁-C₈)Alkyl, (C₁-C₈)Alkoxy, (C₁-C₈)Alkanoyl, (C₁-C₈)Alkanoyloxy, (C₁-C₈)Alkanoylamino, (C₆-C₁₀)Aroylamino, Cyano und/oder Halogen substituiert sind.

Beispiele für Arylsulfonsäuren sind die Benzolsulfonsäure und die Naphthalinsulfonsäure.

Die Heteroarylsulfonsäuren weisen neben 4 bis 9 Kohlenstoffatomen noch ein aromatisches Sauerstoff- oder Schwefelatom oder 1 oder 2 aromatische Stickstoffatome auf. Beispiele hierfür sind die Furan- und Thiophensulfonsäuren, die Pyrrol-, Pyridin-, Pyrimidin- und Pyrazin-sulfonsäuren. Auch Sulfonsäuren mit einem zweikernigen Heteroarylrest sind geeignet. Hierfür seien die Benzofuran-, Isobenzofuran-, Benzo[b]thiophen- und Indolsulfonsäuren genannt. Die stickstoffhaltigen Heterocyclen sollen jedoch nicht basisch sein.

Die Aryl- wie auch die Heteroarylsulfonsäuren können substituiert sein. Im Prinzip können alle Substituenten, die keine unerwünschten Reaktionen eingehen, vorkommen. Geeignete Substituenten sind lineare und verzweigte Alkylgruppen mit vorzugsweise nicht mehr als 8 Kohlenstoffatomen, wie Methyl, Ethyl, Propyl, Isopropyl, Butyl, sec.-Butyl, Isobutyl und tert.-Butyl. Die Alkylgruppen können fluoriert, bevorzugt auch perfluoriert, sein. Von den perfluorierten Alkylresten sind Trifluormethyl und Perfluorbutyl besonders geeignet. Weitere geeignete Substituenten sind (C₁-C₈)Alkoxy, (C₁-C₈)Alkoxy-(C₁-C₈)alkoxy, (C₁-C₈)Alkanoyl, (C₁-C₈)Alkanoyloxy, (C₁-C₈)Alkanoylamino, (C₆-C₁₀)Aryl, (C₆-C₁₀)Aryloxy, (C₆-C₁₀)Aryl-(C₁-C₈)alkoxy, (C₆-C₁₁)Aroylamino, (C₆-C₁₁)Aroylamino-(C₁-C₈)alkyl, Cyano und Halogen. Die Substituenten können mehrfach auftreten. Unabhängig davon können verschiedene Substituenten nebeneinander vorhanden sein. Bevorzugte Substituenten sind (C₁-C₄)Alkyl, (C₁-C₄)Alkoxy und Halogen.

Ein Sulfonsäureester aus 3 mol (Hetero)arylsulfonsäure und 1 mol 2,4,6-Tris-(2-hydroxy-ethoxy-[1,3,5]triazin ist im allgemeinen bevorzugt gegenüber einem Sulfonsäureester aus 2 mol (Hetero)arylsulfonsäure und 1 mol 2,4,6-Tris-(2-hydroxy-ethoxy-[1,3,5]triazin. Ein solches, niedriger verestertes Produkt kann jedoch in speziellen Fällen eine bessere Löslichkeit aufweisen.

Besonders geeignete (Hetero)arylsulfonsäuren sind Benzol-, 3-Perfluoroctyl-benzol-, 4-Trifluormethyl-benzol-, 4-Perfluorbutyl-benzol-, Toluol-4-, 4-Bromo-benzol-, 4-Cyano-benzol-, 4-tert.-Butyl-benzol-, 2,4,5-Trimethyl-benzol-, 3,4-Dichlor-benzolsulfonsäure und 5-Benzoylaminomethyl-thiophen-2-sulfonsäure.

Die mit den Sulfonsäuren gebildeten Ester sind besonders geeignet, weil sie mit einer hohen Quantenausbeute photolysiert werden können, gleichzeitig aber auch eine ausreichende thermische Stabilität besitzen. Die bei der Photolyse gebildete Säure ist stark genug, um die Vernetzung der im erfindungsgemäßen Gemisch enthaltenen vernetzbaren Komponenten b) zu bewirken.

Die Herstellung der in dem erfindungsgemäßen Gemisch eingesetzten, mehrfunktionellen Sulfonsäureester ist an sich bekannt. Als Ausgangsmaterialien dienen dabei in erster Linie die entsprechenden Sulfonsäurechloride. Verfahren zu Herstellung von aromatischen Sulfonsäureestern werden beispielsweise von F. Muth in: Houben-Weyl-Müller, Methoden der organischen Chemie, Bd. IX, S. 633 (und dort zitierte Literatur), Thieme-Verlag, 4. Aufl., Stuttgart 1955, und von S. Pawlenko, a.a.O., Bd. E 11, S. 1084, Thieme-Verlag, 1. Aufl., Stuttgart 1985, sowie in der Patentliteratur an zahlreichen Beispielen beschrieben. Auch die entsprechenden Sulfonsäureanhydride sind als Ausgangsmaterialien geeignet (siehe S. Pawlenko, a.a.O., Bd. E11, S. 1086, Thieme-Verlag, 1. Aufl., Stuttgart 1985, und P. J. Stang, M. Hanack, L. R` Subramaniam, Synthesis, **1982**, 85). Dies gilt insbesondere für die mit Perfluoralkyl-Gruppen substituierten Benzolsulfonsäureanhydride.

Das erfindungsgemäße, strahlungsempfindliche Gemisch zeichnet sich durch eine hohe Empfindlichkeit über einen weiten Spektralbereich aus. Es zeigt eine hohe thermische Stabilität und schafft die Möglichkeit, auch feinste Strukturen einer Vorlage detailgenau wiederzugeben. Die bei der Bestrahlung gebildete Säure wirkt nicht korrodierend, so daß das Gemisch auch auf empfindlichen Substratmaterialien Verwendung finden kann.

Überraschenderweise zeigen die erfindungsgemäßen, positiv arbeitenden, strahlungsempfindlichen Gemische nicht nur eine hohe thermische und Plasmaätzbeständigkeit sondern auch hervorragende lithographische Eigenschaften, die eine Auflösung im Halbmikrometer- und teilweise auch im Sub-Halbmikrometer-Bereich erlauben. Man erhält nach dem bildmäßigen Bestrahlen und anschließendem Entwickeln ein detailgetreues Abbild der Maske. Die Resistfelder weisen steile Flanken auf. In den bestrahlten Bereichen wird die Resistschicht vollständig abgelöst, d. h. es bleiben keinerlei Reste oder Rückstände der Schicht auf dem Substrat zurück. Die bei der Photolyse gebildeten Sulfonsäuren führen zu einer effizienten Spaltung der Resist-Komponente b), was die Herstellung von hochempfindlichen, positiv arbeitenden Gemischen erlaubt.

Mit den erfindungsgemäßen Gemischen hergestellte Aufzeichnungsmaterialien zeigen überraschend eine höchsten Ansprüchen genügende Bilddifferenzierung und, noch überraschender, eine Verbesserung des Kontrasts und des Auflösungsvermögens. Die erfindungsgemäßen Gemische erlauben beispielsweise die Herstellung eines hochempfindlichen positiv arbeitenden Photoresists für energiereiche UV2-Strahlung (z. B. 248 nm).

Da das erfindungsgemäße Gemisch über einen weiten Spektralbereich empfindlich ist, ist zum bildmäßigen Bestrahlen aktinische Strahlung allgemein geeignet. Als aktinische Strahlung soll in diesem Zusammenhang jede Strahlung verstanden werden, deren Energie mindestens der des kurzwelligen sichtbaren Lichts entspricht. Geeignet ist dabei insbesondere UV-Strahlung im Bereich von 190 bis 450 nm, bevorzugt von 200 bis 400 nm, besonders bevorzugt von 200 bis 300 nm, aber auch Elektronen- oder Röntgenstrahlung.

In dem erfindungsgemäßen, strahlungsempfindlichen Gemisch können die bei Bestrahlung Säure bildenden mehrfunktionellen Sulfonsäureester allein oder in Kombination mit anderen Säurebildnern Verwendung finden. Als zusätzliche Säurebildner sind insbesondere die in der gleichzeitig eingereichten deutschen Patentanmeldung P 41 12 973.3 beschriebenen mehrfunktionellen Sulfonsäureester aromatischer Polyhydroxy-Verbindungen geeignet.

Darüber hinaus lassen sich die mehrfunktionellen Sulfonsäureester auch mit Onium-Salzen, Halogenverbindungen, insbesondere Trichlormethyltriazinderivaten oder Trichlormethyloxadiazolderivaten, 1,2-Disulfonen, o-Chinondiazidsulfonylchloriden oder Organometall-Organohalogen-Kombinationen kombinieren. Es kommen aber auch Mischungen mit Bis(sulfonyl)diazomethanen und Sulfonylcarbonyldiazomethanen in Frage. In solchen Gemischen können jedoch die eingangs erwähnten Nachteile wieder auftreten.

Der Anteil an mehrfunktionellen Sulfonsäureestern im erfindungsgemäßen Gemisch liegt im allgemeinen bei 0,5 bis 25 Gew.-%, bevorzugt bei 3 bis 15 Gew.-%, bezogen auf das Gesamtgewicht der Feststoffe im Gemisch.

Als säurespaltbare Verbindungen in dem erfindungsgemäßen, strahlungsempfindlichen Gemisch haben sich vor allem folgende Verbindungsklassen bewährt:
a) solche mit mindestens einer Orthocarbonsäureester- und bzw. oder Carbonsäureamidacetalgruppe, wobei die Verbindungen auch polymeren Charakter haben und die genannten Gruppen in der Hauptkette oder seitenständig auftreten können (DE-A 26 10 842 und 29 28 636),
b) oligomere oder polymere Verbindungen mit wiederkehrenden Acetal- und/oder Ketalgruppen in der Hauptkette (DE-A 23 06 248 und 27 18 254),
c) Verbindungen mit mindestens einer Enolether- oder N-Acyliminocarbonatgruppe (EP-A 0 006 626 und 0 006 627),
d) cyclische Acetale oder Ketale von β-Ketoestern oder -amiden (EP-A 0 202 196),
e) Verbindungen mit Silylethergruppen (DE-A 35 44 165 und 36 01 264),
f) Verbindungen mit Silylenolethergruppen (DE-A 37 30 785 und 37 30 783),
g) Monoacetale bzw. Monoketale von Aldehyden bzw. Ketonen, deren Löslichkeit im Entwickler zwischen 0,1 und 100 g/l beträgt (DE-A 37 30 787),
h) Ether auf der Basis tertiärer Alkohole (US-A 4 603 101) und
i) Carbonsäureester und Carbonate, deren Alkoholkomponente ein tertiärer Alkohol, ein Allylalkohol oder ein Benzylalkohol ist (US-A 4 491 628 und J. M. Fréchet et al., J. Imaging Sci. 30, 59-64 (1986).

Es können auch Mischungen der vorgenannten säurespaltbaren Materialien eingesetzt werden. Bevorzugt ist allerdings ein säurespaltbares Material, das nur einem der obengenannten Typen zugeordnet werden kann, besonders ein solches mit mindestens einer durch Säure spaltbaren C-O-C-Bindung, d. h. daß diejenigen Materialien besonders bevorzugt sind, die den Typen (a), (b), (g) und (i) angehören. Unter Typ (b) sind besonders die polymeren Acetale hervorzuheben; von den säurespaltbaren Materialien des Typs (g) insbesondere diejenigen, die sich von Aldehyden bzw. Ketonen mit einem Siedepunkt über 150 °C, vorzugsweise über 200 °C, ableiten. Besonders bevorzugt sind die in der gleichzeitig eingereichten deutschen Patentanmeldung P 41 12 968.7 beschriebenen N,O-Polyacetale.

Die Verbindung b) bzw. die Kombination von Verbindungen b) hat einen Anteil von 1 bis 50 Gew.-%, bevorzugt 10 bis 40 Gew.-%, am Gesamtgewicht der Feststoffe des strahlungsempfindlichen Gemisches.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel c). Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 300 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den nicht belichteten Bereichen eine Erniedrigung der Löslichkeit gegenüber wäßrigalkalischen Entwicklern erkennen, doch ist ihre Eigenabsorption im Bereich der für die Bestrahlung gewünschten kurzen Wellenlänge unerwünscht hoch.

Novolak-Kondensationsharze können aber in Mischung mit anderen als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Als Bindemittel geeignet sind Homo- oder Copolymere des 4-Hydroxystyrols sowie seiner Alkylderivate, z.B. des 3-Methyl-4-hydroxystyrols, sowie Homo- oder Copolymere anderer Vinylphenole, z. B. des 3-Hydroxystyrols oder der Ester oder Amide von Acrylsäure mit phenolischen Gruppen aufweisenden Aromaten. Als Comonomere können polymerisierbare Verbindungen wie Styrol, Methyl(meth)acrylat oder ähnliche eingesetzt werden.

Gemische mit erhöhter Plasmabeständigkeit werden erhalten, wenn zur Herstellung der Bindemittel Silizium enthaltende Vinylmonomere, z. B. Vinyltrimethylsilan, mitverwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierzu zählen beispielsweise Maleinsäureanhydrid und Maleinsäurehalbester.

Die genannten Bindemittel können auch untereinander gemischt werden, sofern sich dadurch die optische Qualität des strahlungsempfindlichen Gemisches nicht verschlechtert. Bindemittelgemische sind jedoch nicht bevorzugt.

Die Menge des Bindemittels beträgt im allgemeinen 40 bis 95 Gew.-%, insbesondere 50 bis 90 Gew.-%, bezogen auf das Gesamtgewicht der festen Anteile des strahlungsempfindlichen Gemisches.

Die Glasübergangstemperatur des Bindemittels bzw. der Kombination von Bindemitteln liegt vortilhaft bei mindestens 120 °C.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Erfüllung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol und Propylenglykol sowie die davon abgeleiteten Mono- und Dialkylether, besonders die Mono- und Dimethylether sowie die Mono- und Diethylether, Ester abgeleitet aus aliphatischen (C₁-C₆)Carbonsäuren und entweder (C₁-C₈)Alkanolen oder (C₁-C₈)Alkandiolen oder (C₁-C₆)Alkoxy-(C₁-C₈)alkanolen, beispielsweise Ethylacetat, Hydroxyethylacetat, Alkoxyethylacetat, n-Butylacetat, Propylenglykolmonoalkyletheracetat, insbesondere Propylenglykolmethyletheracetat und Amylacetat, Ether, wie Tetrahydrofuran und Dioxan, Ketone, wie Methylethylketon, Methylisobutylketon, Cyclopentanon und Cyclohexanon, N,N-Dialkyl-carbonsäureamide, wie N,N-Dimethylformamid und N,N-Dimethylacetamid, aber auch Hexamethylphosphorsäuretriamid, N-Methyl-pyrrolidin-2-on und Butyrolacton, sowie beliebige Mischungen davon. Besonders bevorzugt von diesen sind die Glykolether, aliphatischen Ester und Ketone.

Letztendlich hängt die Wahl des Lösemittels bzw. Lösemittelgemisches ab von dem angewandten Beschichtungsverfahren, der gewünschten Schichtstärke und den Trocknungsbedingungen. Ebenso müssen die Lösemittel unter den angewendeten Bedingungen gegenüber den übrigen Schichtbestandteilen chemisch inert sein.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, z. B. Glas und Indium-Zinnoxid, ferner Metallplatten und -folien - beispielsweise aus Aluminium, Kupfer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder, zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Bei Silicium- bzw. Siliciumdioxid-Substraten kommen hierfür Haftvermittler vom Aminosilan-Typ, z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Stärke der lichtempfindlichen Schicht hängt vom Verwendungszweck ab. Sie beträgt im allgemeinen zwischen 0,1 und 100 µm, bevorzugt zwischen 0,5 und 10 µm, besonders bevorzugt um 1,0 µm.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt. Danach behandelt man die Schicht mit einer Entwicklerlösung, die die bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können gegebenenfalls geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot-plate bis zu einer Temperatur unterhalb der Fließtemperatur und anschließendes ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht erfolgen.

Verwendung findet das erfindungsgemäße strahlungsempfindliche Gemisch bei der Herstellung von integrierten Schaltungen oder von einzelnen elektrischen Bausteinen mit lithographischen Prozessen, da sie eine hohe Lichtempfindlichkeit aufweisen, besonders bei Bestrahlung mit Licht einer Wellenlänge zwischen 190 bis 300 nm. Da die Gemische bei Belichtung sehr gut ausbleichen, lassen sich feinere Strukturen erzielen als dies mit den bekannten Gemischen möglich ist. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die nachstehend beschriebenen Beispiele illustrieren die Erfindung, sollen aber nicht beschränkend wirken.

### Synthesebeispiel

### 2,4,6-Tris-[2-(toluol-4-sulfonyloxy)-ethoxy]-[1,3,5]triazin

Zu einer Lösung von 20,0 g (76,5 mmol) 2,4,6-Tris-(2-hydroxy-ethoxy)-[1,3,5]triazin und 60,6 g (760 mmol) Pyridin in 400 ml trockenem Acetonitril wurden bei 0 ^{o}C 73,8 g (380 mmol) p-Toluolsulfonsäurechlorid innerhalb von 10 min zugegeben und 4 h bei 0 ^{o}C gerührt. Anschließend wurde das Eisbad entfernt und auf Raumtemperatur erwärmen gelassen. Die Mischung wurde nun langsam und unter ständigem Rühren in 300 ml Eiswasser eingetropft und anschließend mit 400 ml Dichlormethan extrahiert. Die organische Phase wurde mehrmals mit gesättigter, wäßriger Natriumsulfatlösung gewaschen und mit Natriumsulfat getrocknet. Dann wurde das Lösemittel im Vakuum abdestilliert. Der Rückstand wurde in 500 ml Dichlormethan aufgenommen, dreimal mit je 150 ml 3 n wäßriger Salzsäure und zweimal je 150 ml gesättigter, wäßriger Natriumsulfatlösung gewaschen und anschließend mit Magnesiumsulfat getrocknet. Das Lösemittel wurde wiederum im Vakuum abdestilliert. Die letzten flüchtigen Bestandteile wurden im Hochvakuum entfernt, wobei der Rückstand unter Aufschäumen erstarrte. Es wurden 36,5 g (67 %) eines nahezu farblosen Pulvers erhalten.

Die Umkristallisation aus einem Lösemittelgemisch Isopropanol/Aceton ergab ein farbloses Pulver mit einem Schmelzpunkt von 111 ^{o}C.

Die Analyse dieser Verbindung ergab folgende Werte:

| | | | | |
|---|---|---|---|---|
| ber.: | C 49,78 % | H 4,60 % | N 5,81 % | S 13,29 % |
| gef.: | C 49,3 % | H 4,4 % | N 5,6 % | S 13,4 % |

Die Charakterisierung der Sulfonsäureester des 2,4,6-Tris-(2-hydroxy-ethoxy)-[1,3,5]triazins erfolgte durch ¹H- und ¹³C-Hochfeld-Kernresonanzspektren, durch Elementaranalysen sowie dünnschichtchromatographisch (zum Beweis der Abwesenheit von nicht umgesetztem Sulfonsäurechlorid) und ggf. IR-spektroskopisch (zum Beweis der Abwesenheit freier Hydroxygruppen im Produkt).

### Anwendungsbeispiele

Die folgenden Beispiele 1 bis 6 belegen die Eignung des erfindungsgemäßen Gemisches für Aufzeichnungsmaterialien in der Mikrolithographie. Anhand der Vergleichsbeispiele 7 und 8 wird die Überlegenheit der erfindungsgemäßen Gemische gegenüber denen aus dem Stand der Technik gezeigt. Gt steht im Folgenden für Gewichtsteile.

### Beispiel 1

Es wurde eine Beschichtungslösung hergestellt aus
- 4,5 Gt: eines Homopolymeren aus 3-Methyl-4-hydroxystyrol mit einem Erweichungsbereich um 155 ^{o}C und einem mittleren Molekulargewicht um 25.000 [bestimmt durch Gelpermeationschromatographie (GPC)],
- 3,0 Gt: eines N,O-Polyacetals, hergestellt aus Benzaldehyd-dimethylacetal und N-Propylcarbamidsäure-(2-hydroxy-ethyl)-ester analog dem Herstellungsbeispiel 1 der gleichzeitig eingereichten deutschen Patentanmeldung P 41 12 968.7 und
- 0,8 Gt: 2,4,6-Tris-[2-(4-brom-benzolsulfonyloxy)-ethoxy]-[1,3,5]triazin (hergestellt analog dem Synthesebeispiel) in
- 42 Gt: Propylenglykol-monomethylether-acetat.

Die Beschichtungslösung wurde durch einen Filter mit einem Porendurchmesser von 0,2 µm filtriert und auf einen mit einem Haftvermittler (Hexamethyldisilazan) vorbehandelten Wafer aufgeschleudert. Die Schleuderdrehzahl wurden dabei so gewählt, daß nach 1 min Trocknen bei 110 ^{o}C auf der hot plate Schichtdicken um 1,07 µm erhalten wurden.

Das Aufzeichnungsmaterial wurde bildmäßig unter einer Vorlage mit der Strahlung eines KrF-Excimerlasers (248 nm) mit einer Energie von 18 mJ/cm² belichtet und anschließend 1 min bei 80 ^{o}C einem post exposure bake auf einer hot plate unterzogen.

Entwickelt wurde das Aufzeichnungsmaterial mit einer 0,27 n wäßrigen Tetramethylammoniumhydroxid-Lösung.

Nach einer Entwicklungsdauer von 60 s wurde ein fehlerfreies Abbild der Maske mit steilen Resistflanken erhalten, wobei auch Strukturen einer Größe von 0,5 µm und weniger noch detailgetreu aufgelöst waren. Eine rasterelektronenmikroskopische Untersuchung der Flanken der Resistprofile belegte, daß diese praktisch senkrecht zur Substratoberfläche ausgerichtet waren.

Die in den Beispielen 2 bis 6 beschriebenen strahlungsempfindlichen Aufzeichnungsmaterialien ergaben ebenfalls ein fehlerfreies Abbild der Maske mit steilen Resistflanken, wobei auch Strukturen einer Größe von 0,5 µm und weniger noch detailgetreu aufgelöst waren. Die Resistflanken waren in allen Fällen nahezu senkrecht zur Substratoberfläche ausgerichtet. Die Triazin-Derivate wurden gemäß bzw. analog dem Synthesebeispiel hergestellt.

### Beispiel 2

Es wurde eine Beschichtungslösung hergestellt aus
- 4,5 Gt: eines Copolymeren aus 3-Methyl-4-hydroxystyrol/p-Hydroxystyrol (Molverhältnis 75:25) mit einem mittleren Molekulargewicht um 28.000 (GPC),
- 3,0 Gt: eines O,O-Polyacetals, hergestellt aus Benzaldehyd-dimethylacetal und einem Tricyclo[5.2.1.0^{2,6}]decandiyl-bis-methanol ("TCD-Alkohol"-Isomerengemisch), hergestellt analog dem Herstellungsbeispiel 1 der DE-A 37 30 787 und
- 1,0 Gt: 2,4,6-Tris-[2-(toluol-4-sulfonyloxy)-ethoxy]-[1,3,5]triazin, in
- 42 Gt: Propylenglykol-monomethylether-acetat.

Beschichtungs-, Trocknungs- und Entwicklungsbedingungen wie im Beispiel 1.

Das Aufzeichnungsmaterial wurde bildmäßig unter einer Vorlage mit der Strahlung einer Xenon-Quecksilberdampflampe bei 260 nm (mit einem Interferenzfilter) mit einer Energie von 23 mJ/cm² belichtet und 60 s entwickelt.

### Beispiel 3

Ein entsprechend Beispiel 2 hergestellter Wafer wurde unter einer Vorlage mit der Strahlung eines KrF-Excimer-Lasers mit einer Wellenlänge von 248 nm mit einer Energie von 24 mJ/cm² bestrahlt. Nach der Entwicklung wurde ähnlich wie in Beispiel 2 ein originalgetreues Abbild der Vorlage erhalten, in dem auch Strukturen im Sub-Halbmikrometer-Bereich detailgetreu wiedergegeben waren.

### Beispiel 4

Es wurde eine Beschichtungslösung hergestellt aus
- 6,0 Gt: eines Homopolymeren aus 3-Methyl-4-hydroxystyrol mit einem Erweichungsbereich um 155 ^{o}C,
- 2,5 Gt: Terephthaldialdehyd-tetra(phenoxyethyl)di-acetal, hergestellt analog dem Herstellungsbeispiel 1 der DE-A 37 30 787, und
- 0,7 Gt: 2,4,6-Tris-[2-(4-trifluormethyl-benzolsulfonyloxy)-ethoxy]-[1,3,5]triazin, in
- 42 Gt: Propylenglykol-monomethylether-acetat.

Die Lösung wurde wie in Beispiel 1 beschrieben vorbehandelt, aufgeschleudert und 1 min bei 115 ^{o}C getrocknet. Das Aufzeichnungsmaterial wurde bildmäßig unter einer Vorlage mit der Strahlung einer Xenon-Quecksilberdampflampe bei 260 nm (Interferenzfilter) mit einer Energie von 27 mJ/cm² belichtet und 1 min bei 75 ^{o}C getrocknet. Entwickelt wurde wie in Beispiel 1, wobei die belichteten Bereiche innerhalb von 60 s rückstandsfrei abgelöst wurden.

### Beispiel 5

Es wurde eine Beschichtungslösung wie im Beispiel 1 beschrieben hergestellt, jedoch mit der Abweichung, daß anstelle des N,O-Polyacetals als säurelabiler Komponente und des darin verwendeten 2,4,6-Tris-[2-(4-brom-benzolsulfonyloxy)-ethoxy]-[1,3,5]triazins als photoaktivem Säurebildner
- 3,0 Gt: eines N,O-Polyacetals, hergestellt aus Benzaldehyd-dimethylacetal und N-Hexyl-carbamidsäure-(2-hydroxy-ethyl)-ester und
- 0,8 Gt: 2,4,6-Tris-[2-(4-brom-benzolsulfonyloxy)-ethoxy]-[1,3,5]triazin
eingesetzt wurden. Die Lösung wurde wie in Beispiel 1 vorbehandelt, aufgeschleudert, getrocknet und das Aufzeichnungsmaterial bildmäßig mit einer Energie von 17 mJ/cm² belichtet. Nach dem post exposure bake wurde wie in Beispiel 1 entwickelt, wobei die belichteten Bereiche innerhalb von 55 s rückstandsfrei abgelöst wurden.

### Beispiel 6

Es wurde eine Beschichtungslösung wie im Beispiel 2 hergestellt, jedoch mit der Abweichung, daß 0,8 Gt 2,4,6-Tris-[2-(3,5-bis-trifluormethyl-benzolsulfonyloxy)-ethoxy]-[1,3,5]triazin anstelle des dort als photoaktiver Säurebildner verwendeten 2,4,6-Tris-[2-(toluol-4-sulfonyloxy)-ethoxy]-[1,3,5]triazins eingesetzt wurden.

Die Lösung wurde wie in Beispiel 2 beschrieben vorbehandelt, aufgeschleudert, getrocknet und das Aufzeichnungsmaterial bildmäßig mit einer Energie von 26 mJ/cm² belichtet. Nach dem post exposure bake wurde wie in Beispiel 2 entwickelt, wobei die belichteten Bereiche innerhalb von 70 s rückstandsfrei abgelöst wurden.

### Beispiele 7 und 8 (Vergleichsbeispiele)

Die Resistformulierung des Beispiels 2 wurde derart abgeändert, daß die dort verwendete säurebildende Verbindung durch die gleiche Menge an Triphenylsulfoniumhexafluoroantimonat (Beispiel 7) bzw. 2,1-Diazonaphthochinon-4-sulfonsäureester des 4,4'-Bis-(4-hydroxyphenyl)-valeriansäure-(2-ethoxy-ethyl)esters (Beispiel 8) ersetzt wurde.

Nach der Belichtung mit Strahlung einer Wellenlänge von 260 nm (Xenon-Quecksilberdampflampe) und einer Energie von 21 mJ/cm² (Beispiel 7) bzw. 84 mJ/cm² (Beispiel 8) sowie Entwicklung mit 0,27 n wäßriger Tetramethylammoniumhydroxid-Lösung wurden Strukturen, die keine praxisgerechte Bilddifferenzierung zeigten, erhalten. Bei beiden den Fällen traten Strukturen mit sogenanntem "Lackfuß" auf, d. h. Resistreste hafteten in den belichteten Bereichen am Substrat an. Zudem waren Auflösung und Linienprofil unbefriedigend. Selbst unter Verwendung optimierter Bake- und Entwicklungs-Bedingungen konnten keine Resultate erzielt werden, die zu akzeptablen Strukturierungen führten.

## Patentansprüche

1. Ein mit zwei oder drei Aryl- und/oder Heteroarylsulfonsäuren verestertes 2,4,6-Tris-(2-hydroxyethoxy)-[1,3,5]triazin, wobei die Arylsulfonsäuren 6 bis 10 aromatische Kohlenstoffatome und die Heteroarylsulfonsäuren 4 bis 9 aromatische Kohlenstoffatome und entweder 1 aromatisches Sauerstoff- oder Schwefelatom oder 1 bis 2 aromatische Stickstoffatome aufweisen und wobei die Aryl- oder Heteroarylsulfonsäuren gegebenenfalls mit (C₁-C₈)Alkyl, (C₁-C₈)Alkoxy, (C₁-C₈)Alkanoyl, (C₁-C₈)Alkanoyloxy, (C₁-C₈)Alkanoylamino, (C₆-C₁₀)Aroylamino, Cyano und/oder Halogen substituiert sind.

2. Positiv arbeitendes strahlungsempfindliches Gemisch gemäß Anspruch 1 mit
a) einer Verbindung, die unter Einwirkung von aktinischer Strahlung eine starke Säure bildet,
b) einer Verbindung mit mindestens einer durch Säure spaltbaren C-O-C-oder C-O-Si-Bindung und
c) einem in Wasser unlöslichen, in wäßrig-alkalischen Lösungen löslichen oder zumindest quellbaren polymeren Bindemittel,
dadurch gekennzeichnet, daß die Verbindung a) ein mit zwei oder drei Aryl- und/oder Heteroarylsulfonsäuren verestertes 2,4,6-Tris-(2-hydroxy-ethoxy)-[1,3,5]triazin ist, wobei die Arylsulfonsäuren 6 bis 10 aromatische Kohlenstoffatome und die Heteroarylsulfonsäuren 4 bis 9 Kohlenstoffatome und entweder 1 aromatisches Sauerstoff- oder Schwefelatom oder 1 oder 2 aromatische Stickstoffatome aufweisen.

3. Positiv arbeitendes strahlungsempfindliches Gemisch gemäß Anspruch 2, dadurch gekennzeichnet, daß die Aryl- oder Heteroarylsulfonsäuren mit (C₁-C₈)Alkyl, (C₁-C₈)Alkoxy, (C₁-C₈)Alkanoyl, (C₁-C₈)Alkanoyloxy, (C₁-C₈)Alkanoylamino, (C₆-C₁₀)Aloyl-amino, Cyano und/oder Halogen substituiert sind.

4. Positiv arbeitendes strahlungsempfindliches Gemisch gemäß Anspruch 2 oder 3, dadurch gekennzeichnet, daß die Verbindung a) unter Einfluß von Strahlung der Wellenlänge 190 bis 450 nm Säure bildet.

5. Positiv arbeitendes strahlungsempfindliches Gemisch gemäß einem oder mehreren der Ansprüche 2 bis 4, dadurch gekennzeichnet, daß das Bindemittel c) phenolische Hydrorygruppen enthält.

6. Positiv arbeitendes strahlungsempfindliches Gemisch gemäß Anspruch 5, dadurch gekennzeichnet, daß das Bindemittel ein gegebenenfalls substituiertes Poly(hydroxystyrol) ist.

7. Positiv arbeitendes strahlungsempfindliches Gemisch gemäß Anspruch 5, dadurch gekennzeichnet, daß das Bindemittel ein Polymer ist mit einer (Meth)acrylsäure, die mit einer Verbindung verestert ist, die neben der zur Esterbildung benötigten Hydroxygruppe freie; phenolische Hydroxygruppen aufweist.

8. Positiv arbeitendes strahlungsempfindliches Gemisch gemäß einem oder mehreren der Ansprüche 5 bis 7, dadurch gekennzeichnet, daß das Bindemittel c) für Strahlung der Wellenlänge um 248 nm eine Extinktion von weniger als 0,5 µm⁻¹ aufweist.

9. Positiv arbeitendes strahlungsempfindliches Gemisch gemäß einem oder mehreren der Ansprüche 2 bis 8, dadurch gekennzeichnet, daß das Bindemittel c) einen Anteil von 40 bis 95 Gew.-% am Gesamtgewicht der Feststoffe des strahlungsempfindlichen Gemisches hat.

10. Positiv arbeitendes strahlungsempfindliches Gemisch gemäß einem oder mehreren der Ansprüche 2 bis 9, dadurch gekennzeichnet, daß die durch Säure spaltbare Verbindung b) einen Anteil von 1 bis 50 Gew.-% am Gesamtgewicht der Feststoffe des strahlungsempfindlichen Gemisches hat.

11. Positiv arbeitendes strahlungsempfindliches Gemisch gemäß einem oder mehreren der Ansprüche 2 bis 9, dadurch gekennzeichnet, daß die durch Säure spaltbare Verbindung b) einen Anteil von 5 bis 40 Gew.-% am Gesamtgewicht der Feststoffe des strahlungsempfindlichen Gemisches hat.

12. Positiv arbeitendes strahlungsempfindliches Gemisch gemäß Anspruch 8, dadurch gekennzeichnet, daß das Bindemittel c) für Strahlung der Wellenlänge um 248 nm eine Extinktion von weniger als 0,3 µm⁻¹ aufweist.

13. Positiv arbeitendes strahlungsempfindliches Gemisch gemäß einem oder mehreren der Ansprüche 2 bis 12, dadurch gekennzeichnet, daß die säurebildende Verbindung a) einen Anteil von 0,5 bis 25 Gew.-% am Gesamtgewicht der Feststoffe des strahlungsempfindlichen Gemisches hat.

14. Positiv arbeitendes strahlungsempfindliches Aufzeichungsmaterial, im wesentlichen bestehend aus einem Träger und einer darauf befindlichen strahlungsempfindlichen Schicht, dadurch gekennzeichnet, daß die Schicht aus einem positiv arbeitenden Gemisch gemäß einem oder mehreren der Ansprüche 2 bis 13 besteht.

15. Positiv arbeitendes strahlungsempfindliches Aufzeichnungsmaterial gemäß Anspruch 14, dadurch gekennzeichnet, daß die strahlungsempfindliche Schicht bei einer Dicke von 1,0 µm für Strahlung der Wellenlänge 248 nm eine Extinktion von weniger als 0,75 aufweist.

16. Positiv arbeitendes strahlungsempfindliches Aufzeichnungsmaterial gemäß Anspruch 14, durch gekennzeichnet, daß die strahlungsempfindliche Schicht bei einer Dicke von 1,0 µm für Strahlung der Wellenlänge 248 nm eine Extinktion von weniger als 0,50 aufweist.

17. Positiv arbeitendes strahlungsempfindliches Aufzeichnungsmaterial gemäß Anspruch 14, dadurch gekennzeichnet, daß die strahlungsempfindliche Schicht bei einer Dicke von 1,0 µm für Strahlung der Wellenlänge 248 nm eine Extinktion von weniger als 0,35 aufweist.

## Claims

1. A 2,4,6-tris-(2-hydroxyethoxy)-[1,3,5]triazine esterified with two or three aryl- and/or heteroarylsulfonic acids, the arylsulfonic acids having 6 to 10 aromatic carbon atoms and the heteroarylsulfonic acids having 4 to 9 aromatic carbon atoms and either 1 aromatic oxygen or sulfur atom or 1 to 2 aromatic nitrogen atoms and the aryl- or heteroarylsulfonic acids optionally being substituted by (C₁-C₈)alkyl, (C₁-C₈)alkoxy, (C₁-C₈)alkanoyl, (C₁-C₈)alkanoyloxy, (C₁-C₈)alkanoyl-amino, (C₆-C₁₀)aroylamino, cyano and/or halogen.

2. A positive-working radiation-sensitive mixture as claimed in claim 1 with
a) a compound which generates a strong acid under the action of actinic radiation,
b) a compound having at least one acid-cleavable C-O-C or C-O-Si bond and
c) a polymeric binder which is insoluble in water and soluble or at least swellable in aqueous alkaline solutions,
wherein the compound a) is a 2,4,6-tris-(2-hydroxy-ethoxy)-[1,3,5]triazine esterified with two or three aryl- and/or heteroarylsulfonic acids, the arylsulfonic acids having 6 to 10 aromatic carbon atoms and the heteroarylsulfonic acids having 4 to 9 carbon atoms and either 1 aromatic oxygen or sulfur atom or 1 or 2 aromatic nitrogen atoms.

3. A positive-working radiation-sensitive mixture as claimed in claim 2, wherein the aryl- or heteroarylsulfonic acids are substituted by (C₁-C₈)alkyl, (C₁-C₈)alkoxy, (C₁-C₈)alkanoyl, (C₁-C₈)alkanoyloxy, (C₁-C₈)alkanoylamino, (C₆-C₁₀)aroylamino, cyano and/or halogen.

4. A positive-working radiation-sensitive mixture as claimed in claim 2 or 3, wherein the compound a) generates acid under the action of radiation of 190 to 450 nm wavelength.

5. A positive-working radiation-sensitive mixture as claimed in one or more of claims 2 to 4, wherein the binder c) contains phenolic hydroxy groups.

6. A positive-working radiation-sensitive mixture as claimed in claim 5, wherein the binder is a substituted or unsubstituted poly(hydroxystyrene).

7. A positive-working radiation-sensitive mixture as claimed in claim 5, wherein the binder is a polymer containing a (meth)acrylic acid esterified with a compound which, in addition to the hydroxy group required for ester formation, contains free, phenolic hydroxy groups.

8. A positive-working radiation-sensitive mixture as claimed in one or more of claims 5 to 7, wherein the binder c) has an extinction of less than 0.5 µm⁻¹ for radiation of wavelength around 248 nm.

9. A positive-working radiation-sensitive mixture as claimed in one or more of claims 2 to 8, wherein the content of the binder c) is 40 to 95% by weight of the total weight of the solids in the radiation-sensitive mixture.

10. A positive-working radiation-sensitive mixture as claimed in one or more of claims 2 to 9, wherein the content of the acid-cleavable compound b) is 1 to 50% by weight of the total weight of the solids in the radiation-sensitive mixture.

11. A positive-working radiation-sensitive mixture as claimed in one or more of claims 2 to 9, wherein the content of the acid-cleavable compound b) is 5 to 40% by weight of the total weight of the solids in the radiation-sensitive mixture.

12. A positive-working radiation-sensitive mixture as claimed in claim 8, wherein the binder c) has an extinction of less than 0.3 µm⁻¹ for radiation of wavelength around 248 nm.

13. A positive-working radiation-sensitive mixture as claimed in one or more of claims 2 to 12, wherein the content of the acid-generating compound a) is 0.5 to 25% by weight of the total weight of the solids in the radiation-sensitive mixture.

14. A positive-working radiation-sensitive recording material, essentially composed of a support and a radiation-sensitive layer located thereon, wherein the layer is composed of a positive-working mixture as claimed in one or more of claims 2 to 13.

15. A positive-working radiation-sensitive recording material as claimed in claim 14, wherein the radiation-sensitive layer at a thickness of 1.0 µm has an extinction of less than 0.75 for radiation of wavelength 248 nm.

16. A positive-working radiation-sensitive recording material as claimed in claim 14, wherein the radiation-sensitive layer at a thickness of 1.0 µm has an extinction of less than 0.50 for radiation of wavelength 248 nm.

17. A positive-working radiation-sensitive recording material as claimed in claim 14, wherein the radiation-sensitive layer at a thickness of 1.0 µm has an extinction of less than 0.35 for radiation of wavelength 248 nm.

## Revendications

1. 2,4,6-Tris-(2-hydroxyéthoxy)-[1,3,5]triazine estérifiée avec deux ou trois acides aryl- et/ou hétéroarylsulfoniques, dans laquelle les acides arylsulfoniques présentent de 6 à 10 atomes de carbone aromatiques et les acides hétéroarylsulfoniques de 4 à 9 atomes de carbone aromatiques et soit 1 un atome d'oxygène ou un atome de soufre aromatique ou 1 à 2 atomes d'azote aromatiques et dans laquelle les acides aryl- ou hétéroarylsulfoniques sont éventuellement substitués par un groupe alkyle en C₁-C₈, alcoxy en C₁-C₈, alcanoyle en C₁-C₈, alcanoyloxy en C₁-C₈, alcanoylamino en C₁-C₈, aroylamino en C₆-C₁₀, cyano et/ou un atome d'halogène.

2. Composition sensible au rayonnement travaillant en positif selon la revendication 1 avec
a) un composé qui forme un acide fort sous l'action de rayonnement actinique,
b) un composé avec au moins une liaison C-O-C ou C-O-Si clivable par un acide et
c) un liant polymère insoluble dans l'eau, soluble ou au moins capable de gonfler dans des solutions aqueuses alcalines,
caractérisée en ce que le composé a) est une 2,4,6-tris-(2-hydroxyéthoxy)-[1,3,5]triazine estérifiée avec deux ou trois acides aryl- et/ou hétéroarylsulfoniques, dans laquelle les acides arylsulfoniques présentent de 6 à 10 atomes de carbone aromatiques et les acides hétéroarylsulfoniques de 4 à 9 atomes de carbone aromatiques et soit 1 un atome d'oxygène ou un atome de soufre aromatique ou 1 à 2 atomes d'azote aromatiques.

3. Composition sensible au rayonnement travaillant en positif selon la revendication 2, caractérisée en ce que les acides aryl- ou hétéroarylsulfoniques sont substitués par un groupe alkyle en C₁-C₈, alcoxy en C₁-C₈, alcanoyle en C₁-C₈, alcanoyloxy en C₁-C₈, alcanoylamino en C₁-C₈, aroylamino en C₆-C₁₀, cyano et/ou un atome d'halogène.

4. Composition sensible au rayonnement travaillant en positif selon la revendication 2 ou 3, caractérisée en ce que, le composé a) forme un acide sous l'influence de rayonnement de longueur d'onde de 190 à 450 nm.

5. Composition sensible au rayonnement travaillant en positif selon une ou plusieurs des revendications 2 à 4, caractérisée en ce que, le liant c) contient des groupes hydroxy de type phénolique.

6. Composition sensible au rayonnement travaillant en positif selon la revendication 5, caractérisée en ce que, le liant est un poly(hydroxystyrène) éventuellement substitué.

7. Composition sensible au rayonnement travaillant en positif selon la revendication 5, caractérisée en ce que, le liant est un polymère avec un acide (méth)acrylique, qui est estérifié avec un composé qui présente en plus du groupe hydroxy nécessaire à l'estérification, des groupes hydroxy libres de type phénolique.

8. Composition sensible au rayonnement travaillant en positif selon une ou plusieurs des revendications 5 à 7, caractérisée en ce que, le liant c) présente pour l'exposition au rayonnement de la longueur d'onde de 248 nm un coefficient d'extinction de moins de 0,5 µm⁻¹.

9. Composition sensible au rayonnement travaillant en positif selon une ou plusieurs des revendications 2 à 8, caractérisée en ce que, le liant c) a un titre de 40 à 95% en poids par rapport au poids total des matières solides de la composition sensible au rayonnement.

10. Composition sensible au rayonnement travaillant en positif selon une ou plusieurs des revendications 2 à 9, caractérisée en ce que, le composé b) clivable par un acide a un titre de 1 à 50% en poids par rapport au poids total des matières solides de la composition sensible au rayonnement.

11. Composition sensible au rayonnement travaillant en positif selon une ou plusieurs des revendications 2 à 9, caractérisée en ce que, le composé b) clivable par un acide présente une teneur de 5 à 40% en poids du poids total des matières solides de la composition sensible au rayonnement.

12. Composition sensible au rayonnement travaillant en positif selon la revendication 8, caractérisée en ce que le liant c) présente pour le rayonnement de longueur d'onde de 248 nm un coefficient d'extinction de moins de 0,3 µm⁻¹.

13. Composition sensible au rayonnement travaillant en positif selon une ou plusieurs des revendications 2 à 12, caractérisée en ce que, le composé a) source d'acide présente une teneur de 0,5 à 25% en poids du poids total des matières solides de la composition sensible au rayonnement.

14. Matériel d'enregistrement sensible au rayonnement travaillant en positif, constitué essentiellement d'un support et d'une couche sensible au rayonnement déposée dessus, caractérisé en ce que la couche est constituée d'une composition travaillant en positif selon une ou plusieurs des revendications 2 à 13.

15. Matériel d'enregistrement sensible au rayonnement travaillant en positif selon la revendication 14, caractérisé en ce que la couche sensible au rayonnement présente pour une épaisseur de 1,0 µm un coefficient d'extinction inférieur à 0,75 pour le rayonnement de longueur d'onde 248 nm.

16. Matériel d'enregistrement sensible au rayonnement travaillant en positif selon la revendication 14, caractérisé en ce que la couche sensible au rayonnement présente pour une épaisseur de 1,0 µm un coefficient d'extinction inférieur à 0,50 pour le rayonnement de longueur d'onde 248 nm.

17. Matériel d'enregistrement sensible au rayonnement travaillant en positif selon la revendication 14, caractérisé en ce que la couche sensible au rayonnement présente pour une épaisseur de 1,0 µm un coefficient d'extinction inférieur à 0,35 pour le rayonnement de longueur d'onde 248 nm.
